# EUROPEAN PATENT APPLICATION

(11) **EP 4 520 344 A1**
(43) Date of publication of application: **12.03.2025**
(21) Application number: 23799279.7
(22) Date of filing: 05.05.2023
(51) Int. Cl.: A61K 38/05, A61K 9/19, A61P 1/16

(54) **DIPEPTIDE DERIVATIVE COMPOSITION, PREPARATION METHOD THEREFOR, AND USE THEREOF**

(30) Priority: 06.05.2022 CN 202210532274
(71) Applicant: Beijing Continent Pharmaceuticals Co., Ltd., Beijing 101300 (CN)
(72) Inventor: ZHANG, Li, Beijing 100102 (CN); LI, Yanjie, Beijing 100102 (CN); LIU, Hong, Beijing 100102 (CN); LING, Sa, Beijing 100102 (CN); WU, Haiyan, Beijing 100102 (CN)
(74) Representative: Gille Hrabal Partnerschaftsgesellschaft mbB Patentanwälte
(86) International application number: PCT/CN2023/092238
(87) International publication number: WO 2023/213293

(57) **Abstract**

The present invention relates to a dipeptide derivative composition, a preparation method therefor, and use thereof. The composition comprises (a) a compound represented by formula I, (b) glycine, and (c) an antioxidant. The composition has good auxiliary material compatibility. A freeze-dried formulation prepared from the composition has low impurity content and features good stability under high humidity, strong light, and low temperature storage conditions.

## Description

### DIPEPTIDE DERIVATIVE COMPOSITION, PREPARATION METHOD THEREFOR, AND USE THEREOF

The present application claims priority to the prior application with the patent application No. 202210532274.X and entitled "DIPEPTIDE DERIVATIVE COMPOSITION, PREPARATION METHOD THEREFOR, AND USE THEREOF" filed with the China National Intellectual Property Administration on May 6, 2022, which is incorporated herein by reference in its entirety.

### TECHNICAL FIELD

The present disclosure relates to the field of pharmaceutical formulations, in particular to a dipeptide derivative composition, a preparation method therefor, and use thereof.

### BACKGROUND

Liver failure refers to severe liver damage caused by various factors, leading to serious impairment or decompensation of the liver's functions such as synthesis, detoxification, excretion, biotransformation, and the like. Clinically, it is manifested as a group of syndromes including coagulation mechanism disorders, jaundice, hepatic encephalopathy, dehydration, and the like. The fatality rate of liver failure is extremely high.

The patent applications CN201110025509.8 and CN201110025516.8 disclose a dipeptide derivative useful for the treatment of liver failure, the structure of which is shown below:

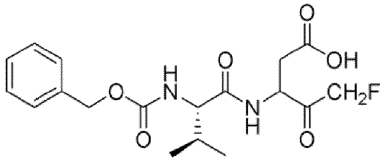

The chemical name of the dipeptide derivative is 3-(2-benzyloxycarbonylamino-3-methylbutanamido)-5-fluoro-4-oxo-pentanoic acid (F573). The dipeptide derivative can remarkably inhibit or reverse liver failure, has remarkable efficacy on liver failure, and has no significant toxicity on cells.

The dipeptide derivative is easy to be destroyed by various enzymes in the oral cavity and gastrointestinal tract environments, and is easy to lose efficacy due to the first pass effect of the liver. Therefore, it is not suitable for the oral dosage form. Moreover, the research has shown that the dipeptide derivative has poor stability in an aqueous solution and cannot resist moist heat sterilization at the same time, and thus there is a need to develop an injectable dosage form with high stability, low impurity content, and low side effects.

### SUMMARY

In order to solve the problems in the prior art, in a first aspect, the present disclosure provides a pharmaceutical composition, which comprises the following components:
(a) a compound of formula I having the following structure: 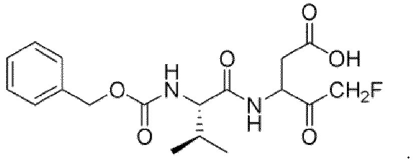
(b) glycine; and
(c) an antioxidant.

According to an embodiment of the present disclosure, the antioxidant is selected from one, two, or more of ascorbic acid, sodium edetate, sodium bisulfite, and sodium metabisulfite.

According to an embodiment of the present disclosure, the composition optionally further comprises component (d) a pH regulator; in some embodiments, the pH regulator is a basic reagent selected from one, two, or more of sodium hydroxide, potassium hydroxide, sodium bicarbonate, potassium bicarbonate, and disodium hydrogen phosphate.

According to an embodiment of the present disclosure, the component (a) accounts for about 0.5% to about 10.0% (w/w), for example, 0.5%, 1.5%, 2.0%, 2.5%, 3.0%, 3.5%, 4.0%, 4.5%, 5.0%, 6.0%, 7.0%, 8.0%, 9.0%, or 10.0% (w/w), of a total amount of the composition. Preferably, the component (a) accounts for about 2.0% to about 5.0% (w/w) of the total amount of the composition.

According to an embodiment of the present disclosure, the component (b) accounts for about 0.5% to about 15.0% (w/w), for example, 0.5%, 1.5%, 2.0%, 2.5%, 3.0%, 3.5%, 4.0%, 4.5%, 5.0%, 5.5%, 6.0%, 6.5%, 7.0%, 7.5%, 8.0%, 8.5%, 9.0%, 9.5%, 10.0%, 11.0%, 12.0%, 13.0%, 14.0%, or 15.0% (w/w), of the total amount of the composition. Preferably, the component (b) accounts for about 3.0% to about 10.0% (w/w) of the total amount of the composition.

According to an embodiment of the present disclosure, the component (c) accounts for about 0.01% to about 0.50% (w/w), for example, 0.01%, 0.05%, 0.10%, 0.15%, 0.20%, 0.25%, 0.30%, 0.40%, or 0.50% (w/w), of the total amount of the composition. Preferably, the component (c) accounts for about 0.05% to about 0.30% (w/w) of the total amount of the composition.

According to an embodiment of the present disclosure, the composition has a pH value greater than 7.0. In some embodiments, the pH value of the composition is in a range selected from 7.0-9.0, such as 7.0, 7.1, 7.2, 7.3, 7.4, 7.5, 7.6, 7.7, 7.8, 7.9, 8.0, 8.1, 8.2, 8.3, 8.4, 8.5, 8.6, 8.7, 8.8, 8.9, or 9.0, preferably 7.5-8.5.

According to an embodiment of the present disclosure, the composition is a lyophilized powder injection.

In a second aspect, the present disclosure provides a preparation method for the pharmaceutical composition, which comprises the following steps:
(a1) adding formula amounts of the antioxidant and glycine, optionally adding the pH regulator, and mixing well; and
(a2) adding a formula amount of the compound of formula I to the solution obtained in step (a1) for dissolving.

According to an embodiment of the present disclosure, the preparation method for the pharmaceutical composition further comprises the following step:
(a3) sterilizing, filtering, and lyophilizing the liquid obtained in step (a2).

According to an embodiment of the present disclosure, in step (a1), the formula amounts of the antioxidant and glycine are added to the pH regulator; preferably, the pH regulator is in the form of an aqueous solution having a concentration of 0.5-3 mol/L, such as 0.5 mol/L, 1.0 mol/L, 1.5 mol/L, 2.0 mol/L, or 3.0 mol/L; more preferably, the pH regulator is an aqueous sodium bicarbonate solution at 1 mol/L; in some embodiments, the liquid obtained in step (a1) has a pH value greater than 7.0, preferably a pH value in a range of 7.0-9.0, and more preferably, 7.5-8.5.

According to an embodiment of the present disclosure, in step (a2), a temperature of the solution obtained in step (a1) is controlled to be 8-15 °C, and then the formula amount of the compound of formula I is added; in some embodiments, the compound of formula I is first sieved with a 80- to 200-mesh sieve (e.g., which may be selected from a 100-mesh sieve) and then added to the solution obtained in step (a1).

According to an embodiment of the present disclosure, steps (a1) and (a2) are performed under nitrogen atmosphere.

According to an embodiment of the present disclosure, in step (a3), the sterilization and filtration are performed by using a microfiltration membrane. Preferably, the microfiltration membrane is a polyethersulfone microfiltration membrane.

According to a preferred embodiment of the present disclosure, the preparation method for the pharmaceutical composition comprises the following steps:
weighing out formula amounts of sodium metabisulfite and glycine, adding sodium metabisulfite and glycine to a sodium bicarbonate solution, stirring until completely dissolved, and adjusting the pH value to 7.0-9.0; controlling the temperature of the solution to be 8-15 °C; adding a formula amount of F573, and stirring for 30-50 min until dissolved; performing the whole solution preparation process under nitrogen atmosphere, followed by sterilization, filtration, and bottling; adding a blooming butyl rubber stopper to an appropriate height at the same time; and performing lyophilization. Preferably, the preparation method further comprises, after the lyophilization, stoppering in a box, taking out from the box, capping, visual inspection, labeling, packaging, sending samples, and warehousing the finished product after passing the inspection.

In a third aspect, the present disclosure provides use of the pharmaceutical composition for manufacturing a medicament for the prevention or treatment of liver failure.

According to an embodiment of the present disclosure, the liver failure comprises: acute liver failure, subacute liver failure, acute-on-chronic liver failure, and chronic liver failure.

According to an embodiment of the present disclosure, the medicament is further used for increasing the survival rate of patients with liver failure, and/or for improving liver function indexes in the patients with liver failure. In some embodiments, improving the liver function indexes comprises: decreasing alanine aminotransferase (ALT), decreasing aspartate aminotransferase (AST), and/or decreasing total bilirubin (TBil).

### Beneficial Effects

The present disclosure provides a stable composition of a dipeptide derivative of formula I, which has good auxiliary material compatibility, and a lyophilized formulation prepared from the composition has low impurity content and features good stability under high-humidity, strong light, and low-temperature storage conditions.

### DETAILED DESCRIPTION

The technical solutions of the present disclosure will be further described in detail with reference to the following specific examples. It will be appreciated that the following examples are merely exemplary illustrations and explanations of the present disclosure and should not be construed as limiting the claimed scope of the present disclosure. All techniques implemented on the basis of the content described above of the present disclosure are encompassed within the claimed scope of the present disclosure.

Unless otherwise stated, the starting materials and reagents used in the following examples are all commercially available products or can be prepared by using known methods.

Reagent: 3-(2-benzyloxycarbonylamino-3-methyl-butanamido)-5-fluoro-4-oxo-pentanoic acid (abbreviated as F573, available from Beijing Continent Pharmaceuticals Co., Ltd.), which has the following structure:

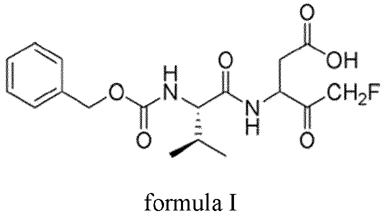

Instruments and equipment: LYO-25 pharmaceutical vacuum freeze dryer; Shimadzu 20A high performance liquid chromatograph; HP1100 high performance liquid chromatograph; Mettler AE240 balance.

### Example 1. Preparation of F573 Injection

### 1.1 For the purpose of preparing F573 injection, screening injection solvents

**Table A-1. Solvent effects**

| Type | Solvent | Amount | Dissolution behavior |
|---|---|---|---|
| Water for injection | Water for injection; adjusting pH to 4.0-5.0 | 2ml:30mg F573 | Turbid |
| Water for injection | Water for injection; adjusting pH to 2.0-2.5 | 2ml:30mg F573 | Turbid |
| Oil for injection | Vegetable oil | 2ml:30mg F573 | Turbid |
| Non-aqueous solvent | Ethanol/water (10%/90%) | 2ml:30mg F573 | Turbid |
| Non-aqueous solvent | Propylene glycol/water (50%/50%) | 2ml:30mg F573 | Turbid |
| Non-aqueous solvent | Propylene glycol/water (80%/20%); adjusting pH to 4.0-5.0 | 2ml:30mg F573 | Clarified |

The results show that F573 was completely dissolved in a solution of propylene glycol/water (80%/20%; pH was adjusted to 4.0-5.0).

### 1.2 Investigation of stability of F573 injection

F573 was dissolved in the solution of propylene glycol/water (80%/20%; pH was adjusted to 4.0-5.0), and the resulting mixture was sterilized at a high temperature of 120 °C for 15 min and then examined (the results are shown in Table A-2 below).

**Table A-2. Investigation results of F573 solution before and after high-temperature sterilization**

| | Content (mg/mL) | Related substances (%) |
|---|---|---|
| Before sterilization | 16.08 | 1.80 |
| After sterilization | 8.82 | 47.22 |

The results show that after the high-temperature sterilization, the sample showed an increase in the related substances of about 50% and a reduction in the F573 content of about 50%. It can be seen that F573 has poor stability in the aqueous solution and cannot resist moist heat sterilization, such that it is not suitable to be developed into small-volume or large-volume injections.

### Example 2. Powder Injection (F573 + Non-Aqueous Solvent Preparation for Use)

An F573 powder injection in composite packaging was prepared, i.e., each set contained 1 vial of sterile powder with 30 mg of main drug and 2 mL of non-aqueous solvent, which was prepared immediately prior to use. An appropriate amount of analgesic (benzyl alcohol) was added as needed, and the F573 starting material was dissolved, crystallized, subjected to carbon removal, dried, etc. by using a 90% ethanol solution as a solvent. The results show that the treatment processes, such as recrystallization, carbon removal, and the like, of the starting material led to a yield of about 50% and may cause problems such as crystal form modification and the like. Therefore, F573 is not suitable to be developed into the dosage form described above.

### Example 3. Research on Lyophilized Composition Formula

### 3.1 Effects of auxiliary materials

In lyophilized formulations, mannitol can serve as a carrier for the formation of a uniform skeleton; while amino acids can not only be used as skeleton agents for the lyophilized formulations, but also be common protein protective agents. A formula of F573 with arginine, glycine, and mannitol and without other auxiliary materials was selected and prepared by adopting the following lyophilization process: F573 was sieved with a 100-mesh sieve for later use; an appropriate amount of sodium bicarbonate was weighed out and prepared into a solution at 1 mol/L for later use; a formula amount of arginine, glycine, or mannitol was weighed out, added to the sodium bicarbonate solution described above, and stirred until completely dissolved; the temperature of the solution was controlled to be 8-15 °C; a formula amount of F573 was added while stirring, and the mixture was stirred for 40 min until dissolved; the whole solution preparation process was performed under nitrogen atmosphere; sterilization and filtration were performed by using a 0.22 µm polyethersulfone microfiltration membrane (filter cartridge) as a terminal filter, and the mixture was bottled; a blooming butyl rubber stopper was added to an appropriate height at the same time; and lyophilization was performed to obtain a block.

The research results are shown in Table B-1 below, and it can be seen that glycine is more suitable as a skeleton agent.

**Table B-1**

| Formula/amount | Appearance | F573% content (labeled amount) | Related substances% |
|---|---|---|---|
| F573 (30g) | Off-white porous block | 100.4 | 2.51 |
| F573 (30 g) + arginine (50 g) | Light yellow porous block | 45.1 | 14.28 |
| F573 (30 g) + glycine (50 g) | Off-white porous block | 92.1 | 2.65 |
| F573 (30 g) + mannitol (50 g) | Off-white porous block; having atrophy phenomenon | 96.5 | 2.65 |

3.2 Test results of compatibility of F573 and auxiliary materials are shown below. F573 was separately combined with glycine and sodium metabisulfite for research, as shown in Tables B-2 and B-3 below. It can be seen that F573 is suitable for combining with glycine and sodium metabisulfite.

**Table B-2. Test results of compatibility of F573 and glycine**

| Condition | Time (days) | Test results | | |
|---|---|---|---|---|
| | | Appearance | Content (labeled amount%) | Related substances% |
| | 0 | White powder | 91.79 | 1.34 |
| 60 °C | 5 | White powder | 104.44 | 1.26 |
| | 10 | White powder | 84.76 | 1.27 |
| Illumination | 5 | White powder | 97.83 | 1.36 |
| | 10 | White powder | 91.71 | 1.51 |
| High humidity | 5 | White powder | 89.54 | 1.41 |
| | 10 | White powder | 106.95 | 1.39 |

**Table B-3. Test results of compatibility of F573 and sodium metabisulfite**

| Condition | Time (day) | Test results | | |
|---|---|---|---|---|
| | | Appearance | Content (labeled amount%) | Related substances% |
| | 0 | White powder | 97.03 | 1.43 |
| 60 °C | 5 | White powder | 91.62 | 1.51 |
| | 10 | White powder | 99.42 | 1.75 |
| Illumination | 5 | White powder | 94.76 | 1.40 |
| | 10 | White powder | 95.88 | 1.63 |
| High humidity | 5 | White powder | 99.67 | 1.13 |
| | 10 | White powder | 99.12 | 1.22 |

### 3.3 Formula optimization experiment

Sodium metabisulfite and glycine were selected as auxiliary materials for the F573 lyophilized formulation, and these 2 factors were investigated to further optimize the formula. For each factor, 3 levels were set, and an orthogonal table L9 (34) was used to arrange 9 formulas for experiments. The results were analyzed by taking the physical appearance, reconstitution time, and pH value of the lyophilized needle as evaluation indexes. The distribution of the experimental factor levels is shown in Table C-1, and the experimental results and statistical analysis are shown in Table C-2 and Table C-3.

**Table C-1. Factor levels**

| Level | Factor A Antioxidant | Factor B Skeleton agent |
|---|---|---|
| 1 | Sodium metabisulfite 0.05% | Glycine 20 mg/injection |
| 2 | Sodium metabisulfite 0.1% | Glycine 35 mg/injection |
| 3 | Sodium metabisulfite 0.2% | Glycine 50 mg/injection |

**Table C-2. Formula optimization test results**

| Test No. | Factor | | | Appearance Score | Reconstitution time (s) | pH | Comprehensive score (Y) |
|---|---|---|---|---|---|---|---|
| | A | B | C | | | | |
| 1 | 1 (0.05%) | 1 (20 mg) | 1 | 1 | 6 | 8.79 | 10 |
| 2 | 1 | 2 (35 mg) | 2 | 4 | 10 | 8.47 | 50.0 |
| 3 | 1 | 3 (50 mg) | 3 | 5 | 25 | 8.25 | 90 |
| 4 | 2 (0.1%) | 1 | 2 | 1 | 7 | 8.71 | 10.6 |
| 5 | 2 | 2 | 3 | 4 | 12 | 8.45 | 53.8 |
| 6 | 2 | 3 | 1 | 5 | 22 | 8.27 | 84.1 |
| 7 | 3 (0.2%) | 1 | 3 | 1 | 7 | 8.66 | 9.7 |
| 8 | 3 | 2 | 1 | 4 | 13 | 8.35 | 54.1 |
| 9 | 3 | 3 | 2 | 5 | 19 | 8.26 | 77.5 |
| Yj1 | 150 | 30.3 | 148.2 | T=∑Yi=439.8 | | | |
| Yj 2 | 148.5 | 157.9 | 138.1 | T²=193424.04 | | | |
| Yj 3 | 141.3 | 251.6 | 153.5 | | | | |
| Yj1² | 22500 | 918.09 | 21963.24 | P=1/9*T²=21491.56 | | | |
| Yj2² | 22052.25 | 24932.41 | 19071.61 | | | | |
| Yj3² | 19965.69 | 63302.56 | 23562.25 | Qr=∑(Yi²)=29806.76 | | | |
| Qj | 21505.98 | 29717.69 | 21532.37 | | | | |
| Sj | 14.42 | 8226.13 | 40.81 | | | | |

| | | | | | | | |
|---|---|---|---|---|---|---|---|
| Note: In the comprehensive evaluation, the appearance accounts for 50%, the reconstitution time accounts for 40%, and the pH value accounts for 10%. | | | | | | | |

**Table C-3. Analysis of variance**

| Variance Source | Deviation from average Sum of squares | Degree of freedom | Variance | F value | Critical value | Significance | Optimal formula |
|---|---|---|---|---|---|---|---|
| B | 8226.13 | 2 | 4113.07 | 276.97 | F0.05 (2, 6)=5.14; F0.01 (2,6)=10.92 | The effect is very remarkable | B3 |
| Error | 89.07 | 6 | 14.85 | | | | |
| Sum | S=8315.2 | 8 | | | | | |

### Example 4. Lyophilized Formulation F-1

### 4.1 Preparation of F-1

Formula of formulation F-1 (amount for 1000 doses):
F573, 30 g;
Glycine, 50 g;
Sodium metabisulfite, 1 g;
Sodium bicarbonate, appropriate amount;
Water for injection added to 1000 mL.

F573 was sieved with a 100-mesh sieve for later use. An appropriate amount of sodium bicarbonate was weighed out and prepared into a solution at 1 mol/L for later use. Additionally, the formula amounts of sodium metabisulfite and glycine were weighed out, added to the sodium bicarbonate solution described above, and stirred until completely dissolved, and the pH value was adjusted to 7.5-8.5; the temperature of the solution was controlled to be 8-15 °C; the formula amount of F573 was added while stirring, and the mixture was stirred for 40 min until dissolved; and the whole solution preparation process was performed under nitrogen atmosphere. The resulting solution was detected, and then filtered after the pH and the content were qualified. Sterilization and filtration were performed by using a polyethersulfone microfiltration membrane (filter cartridge) as a terminal filter, the drug liquid was filled into a pharmaceutical bottle after the visible particles were checked to be qualified, and the deliverable volume was adjusted before bottling; and a butyl rubber stopper was added to an appropriate height at the same time. The sample was then lyophilized, stoppered in a box, taken out from the box, capped, subjected to visual inspection, labeled, packaged, and send for analysis, and the finished product was warehoused after passing the inspection. The finished product was stored at 4-8 °C.

4.2 Three batches of samples prepared according to the preparation process of F-1 described above were taken and investigated for the long-term stability under high-humidity, strong light, and low-temperature storage conditions. The test results are shown below. The results show that each inspection item for F573 for injection complies with the regulations for formulation stability in the pharmacopeia.

**Table D-1. Test results of three batches of samples**

| Batch No. | Test results | | | | | |
|---|---|---|---|---|---|---|
| | Appearance | pH value | Content (labeled amount%) | Related substances% | | Moisture (%) |
| | | | | Maximum impurity% | Total impurities (%) | |
| 20110501 | Off-white porous block | 8.5 | 96.3 | 0.6 | 4.5 | 2.5 |
| 20110502 | Off-white porous block | 8.5 | 97.9 | 0.6 | 4.5 | 2.5 |
| 20110503 | Off-white porous block | 8.8 | 94.4 | 0.5 | 4.0 | 2.6 |

**Table D-2. Stability test results of F573 (20110501) for injection under the 90.0% high humidity (25 °C) condition**

| Days of storage | Shape Appearance/ color | Acidity value | Single maximum impurity (%) | Total impurities (%) | Content (%) | Comprehensive evaluation |
|---|---|---|---|---|---|---|
| 0 | White lyophilized powder | 8.5 | 0.61 | 4.52 | 96.3 | Comply with the standard |
| 5 | White lyophilized powder | 8.5 | 0.77 | 5.34 | 92.7 | Comply with the standard |
| 10 | White lyophilized powder | 8.5 | 0.83 | 5.48 | 92.2 | Comply with the standard |

**Table D-3. Stability test results of F573 (20110501) for injection under the 4500 lx strong light irradiation condition**

| Days of storage | Shape Appearance/ color | Acidity value | Single maximum impurity (%) | Total impurities (%) | Content (%) | Comprehensive evaluation |
|---|---|---|---|---|---|---|
| 0 | White lyophilized powder | 8.5 | 0.61 | 4.52 | 96.3 | Comply with the standard |
| 5 | White lyophilized powder | 8.5 | 0.83 | 5.64 | 92.7 | Comply with the standard |
| 10 | White lyophilized powder | 8.5 | 1.04 | 6.79 | 90.4 | Comply with the standard |

**Table D-4. Long-term test results of F573 (20110501) for injection stored in a refrigerator (2-8 °C)**

| Time (months) | Maximum impurity (%) | Total impurities (%) | Content (%) | Comprehensive evaluation |
|---|---|---|---|---|
| 18 | 0.744 | 4.704 | 95.296 | Comply with the standard |
| 24 | 0.978 | 5.413 | 94.587 | Comply with the standard |

**Table D-5. Long-term test results of F573 (20110502) for injection stored in a refrigerator (2-8 °C)**

| Time (months) | Maximum impurity (%) | Total impurities (%) | Content (%) | Comprehensive evaluation |
|---|---|---|---|---|
| 18 | 0.754 | 4.768 | 95.232 | Comply with the standard |
| 24 | 0.948 | 5.277 | 94.723 | Comply with the standard |

**Table D-6. Long-term test results of F573 (20110503) for injection stored in a refrigerator (2-8 °C)**

| Time (months) | Maximum impurity (%) | Total impurities (%) | Content (%) | Comprehensive evaluation |
|---|---|---|---|---|
| 18 | 0.752 | 4.631 | 95.369 | Comply with the standard |
| 24 | 1.001 | 5.524 | 94.476 | Comply with the standard |

The exemplary embodiments of the present disclosure have been described above. However, the scope of the present disclosure is not limited to the above embodiments. Any modification, equivalent replacement, improvement, and the like made without departing from the spirit and principle of the present disclosure shall fall within the protection scope of the present disclosure.

## Claims

1. A pharmaceutical composition, comprising the following components:
(a) a compound of formula I having the following structure: 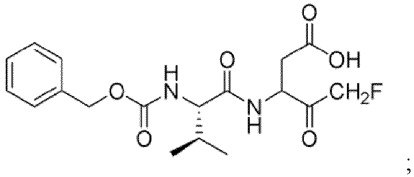
(b) glycine; and
(c) an antioxidant.

2. The pharmaceutical composition according to claim 1, wherein the antioxidant is selected from one, two, or more of ascorbic acid, sodium edetate, sodium bisulfite, and sodium metabisulfite.

3. The pharmaceutical composition according to claim 1, wherein the composition optionally further comprises component (d) a pH regulator; in some embodiments, the pH regulator is a basic reagent selected from one, two, or more of sodium hydroxide, potassium hydroxide, sodium bicarbonate, potassium bicarbonate, and disodium hydrogen phosphate.

4. The pharmaceutical composition according to claim 1, wherein
the component (a) accounts for 0.5% to 10.0% (w/w) of a total amount of the composition; preferably, the component (a) accounts for 2.0% to 5.0% (w/w) of the total amount of the composition;
the component (b) accounts for about 0.5% to about 15.0% (w/w) of the total amount of the composition; preferably, the component (b) accounts for 3.0% to about 10.0% (w/w) of the total amount of the composition;
the component (c) accounts for 0.01% to 0.50% (w/w) of the total amount of the composition; preferably, the component (c) accounts for 0.05% to 0.30% (w/w) of the total amount of the composition.

5. The pharmaceutical composition according to claim 1, wherein the composition has a pH value greater than 7.0, preferably 7.0-9.0, and more preferably 7.5-8.5.

6. The pharmaceutical composition according to claim 1, wherein the composition is a lyophilized powder injection.

7. A preparation method for the pharmaceutical composition according to any one of claims 1-6, comprising the following steps:
(a1) adding formula amounts of the antioxidant and glycine, optionally adding the pH regulator, and mixing well; and
(a2) adding a formula amount of the compound of formula I to the solution obtained in step (a1) for dissolving;
preferably, further comprising the following step:
(a3) sterilizing, filtering, and lyophilizing the liquid obtained in step (a2).

8. The preparation method according to claim 7, wherein
in step (a1), the formula amounts of the antioxidant and glycine are added to the pH regulator;
in step (a2), a temperature of the solution obtained in step (a1) is controlled to be 8-15 °C, and then the formula amount of the compound of formula I is added.

9. The preparation method according to claim 7, comprising the following steps:
weighing out formula amounts of sodium metabisulfite and glycine, adding sodium metabisulfite and glycine to a sodium bicarbonate solution, stirring until completely dissolved, and adjusting the pH value to 7.0-9.0; controlling the temperature of the solution to be 8-15 °C; adding a formula amount of F573, and stirring for 30-50 min until dissolved; performing the whole solution preparation process under nitrogen atmosphere, followed by sterilization, filtration, and bottling; adding a blooming butyl rubber stopper to an appropriate height at the same time; and performing lyophilization.

10. Use of the pharmaceutical composition according to any one of claims 1-6 for manufacturing a medicament for the prevention or treatment of liver failure.

11. The use according to claim 10, wherein the liver failure comprises: acute liver failure, subacute liver failure, acute-on-chronic liver failure, and chronic liver failure; preferably, the medicament is further used for increasing the survival rate of patients with liver failure, and/or for improving liver function indexes in the patients with liver failure; more preferably, improving the liver function indexes comprises: decreasing alanine aminotransferase (ALT), decreasing aspartate aminotransferase (AST), and/or decreasing total bilirubin (TBil).
